# EUROPEAN PATENT APPLICATION

(11) **EP 3 906 964 A1**
(43) Date of publication of application: **10.11.2021**
(21) Application number: 21172829.0
(22) Date of filing: 07.05.2021
(51) Int. Cl.: A61N 1/05, A61B 5/268

(54) **RECESSED ELECTRODES FOR FLEXIBLE SUBSTRATES**

(30) Priority: 08.05.2020 US 202063021799 P
(71) Applicant: Heraeus Deutschland GmbH & Co KG, 63450 Hanau (DE); Heraeus Medical Components, LLC, St. Paul, MN 55127 (US)
(72) Inventor: Stoica, Leonard, 63450 Hanau (DE); Hendricks, Jeffrey, St. Paul, 55127 (US)
(74) Representative: Herzog IP Patentanwalts GmbH

(57) **Abstract**

An implantable medical electrode for stimulation or sensing, wherein the electrode comprises: an electrically insulating substrate having a substrate surface, wherein the substrate surface comprises at least one recess, wherein the at least one recess comprises: a bottom surface arranged at a first distance to the substrate surface and at least one wall connecting the bottom surface to the substrate surface; a conducting element arranged in contact with the bottom surface; a layer of conductive polymer in contact with the conducting element thus forming an electrical connection with the conducting element when a current is applied.

## Description

### BACKGROUND OF THE INVENTION

The present disclosure relates to an implantable medical electrode for electrophysiological applications (e.g., stimulation, sensing, mapping, and ablation) within a body. In particular, the present disclosure relates to the construction of an electrode component of an implantable medical electrical lead.

Implantable biomedical electrodes are a primary component of many medical devices, including cardiac pacemakers and defibrillators, deep brain stimulation devices, cochlear implants, peripheral nerve stimulation devices, spinal cord stimulation devices for pain management, and diagnostic tools. The electrode(s) found on the tip of biomedical leads are placed in contact with the appropriate target tissue and are used to transmit bio-electrical signals to and from the device and target tissue.

A variety of implantable medical devices on the market today utilize conductive electrode coatings comprised of metal oxides or metal nitrides. Depending on how they are deposited, coatings comprised of metal oxides or metal nitrides can have a variety of topographies and morphologies. When used for medical device electrode coatings, metal oxides or metal nitrides are typically formulated with a microscale roughness and/or porosity such that the surface area is significantly increased over that of the uncoated electrode, which lowers the overall electrical impedance. Despite their rough, high surface area topography, however, metal oxide and metal nitride coatings are still mechanically hard compared to the surrounding soft, biological tissue, which is undesirable in the context of a medical device, and particularly a device intended for long-term implantation.

Furthermore, when used with devices that deliver electrostimulation therapies, common metal oxide electrode coatings become increasingly destabilized as the electrode undergoes cycles of biphasic pulse stimulation, due to the build-up of brittle oxide layers at the surface of the electrode. This degradation of the coating presents numerous problems and undesirable qualities for implanted medical device electrodes; these are the potential for tissue injury due to exposure to the delaminated chunks/layers of metal oxide and exposure to potentially harmful non-uniform or higher than usual charge densities caused by the resulting non-uniform electrode surface.

Conductive polymer coatings have overcome some of the drawbacks associated with traditional metal oxide or metal nitride coatings. For example, conductive polymer coatings derived from poly(3,4-ethylenedioxythiophene) (PEDOT) have been employed in medical electrode applications. PEDOT coatings have addressed many of the drawbacks of metal medical device electrodes by providing the metal with significantly improved electrical properties. Specifically, the PEDOT coatings provide a metal medical device electrode with 1 to 3 orders of magnitude decrease in electrode impedance, an increase in charge storage capacity (CSC) often as high as about 1000%, and significantly reduced electrode polarization or peak to peak voltage/current response to a biphasic current or voltage pulse. Cast, dipped, sprayed, or CVD-deposited coatings of PEDOT or PEDOT-derived coatings on metal substrates, however, typically exhibit limited adhesion to metal substrates.

Implantable medical systems that are designed to deliver electrical stimulation, for example, to the brain, cardiac muscle or the spinal cord, and/or to monitor bodily electrical activity, may comprise a flexible substrate. In addition to flexibility, such substrates are also an efficient and promising method to add channel count to a device. These flexible substrates are typically coated post assembly with electrodeposition coatings such as conductive polymers to improve the electrical performance of the implanted device. Devices comprising such substrates are typically implanted via a catheter or guide tube to access the targeted region of the body. These insertion devices are typically a line-to-line fit with the ID of the catheter or insertion tube to the OD of the device. This situation can provide an opportunity to scratch or damage the coating on the electrodes thereby putting the patient's care at risk.

Thus, there is a need in the art for an electrode design that does not suffer from the above-mentioned drawbacks.

### BRIEF SUMMARY OF THE INVENTION

The recessed electrode described herein satisfies this need.

A recessed electrode would have the electrode below the max OD of the device and therefore be protected from the insetion process to result in an undamaged device.

Apart from mechanical protection of the film, the recessed electrodes will allow an extensive charge storage capacitance accumulation on the electrodes (important for neurostimulation applications), but still preventing the lateral film growth, thus maintaining the electrode dimensions to the initial geometrical limits.

A 1^{st} embodiment of the invention is an implantable medical electrode for stimulation or sensing, wherein the electrode comprises: an electrically insulating substrate having a substrate surface, wherein the substrate surface comprises at least one recess, wherein the at least one recess comprises: a bottom surface arranged at a first distance to the substrate surface and at least one wall connecting the bottom surface to the substrate surface; a conducting element arranged in contact with the bottom surface; a layer of conductive polymer in contact with the conducting element thus forming an electrical connection with the conducting element when a current is applied.

In a preferred embodiment of the implantable medical electrode, the layer of conductive polymer has a maximum thickness that is less than or equal to the substrate surface. This preferred embodiment is a 2^{nd} embodiment of the invention, that preferably depends on the 1^{st} embodiment of the invention.

In a preferred embodiment of the implantable medical electrode, the substrate is flexible. This preferred embodiment is a 3^{rd} embodiment of the invention, that preferably depends on any of the 1^{st} to 2^{nd} embodiments of the invention.

In a preferred embodiment of the implantable medical electrode, the layer of conductive polymer protrudes above the substrate surface. This preferred embodiment is a 4^{th} embodiment of the invention, that preferably depends on the 1^{st} embodiment of the invention.

In a preferred embodiment of the implantable medical electrode, the layer of conductive polymer has a charge storage capacitance ranging from 1 to 400 mC/cm². This preferred embodiment is a 5^{th} embodiment of the invention, that preferably depends on any of the 1^{st} to 4^{th} embodiments of the invention.

In a preferred embodiment of the implantable medical electrode, the implantable medical electrode further comprises a connecting element capable of connecting the conducting element to a current source. This preferred embodiment is a 6^{th} embodiment of the invention, that preferably depends on any of the 1^{st} to 5^{th} embodiments of the invention.

In a preferred embodiment of the implantable medical electrode, the layer of conductive polymer comprises PEDOT. This preferred embodiment is a 7^{th} embodiment of the invention, that preferably depends on any of the 1^{st} to 6^{th} embodiments of the invention.

In a preferred embodiment of the implantable medical electrode, the layer of conductive polymer protruding above the substrate surface comprises a pattern of lines. This preferred embodiment is an 8^{th} embodiment of the invention, that preferably depends on the 4^{th} embodiment of the invention.

In a preferred embodiment of the implantable medical electrode, the layer of conductive polymer protruding above the substrate surface also extends laterally beyond the recess. This preferred embodiment is a 9^{th} embodiment of the invention, that preferably depends on the 4^{th} embodiment of the invention.

In a preferred embodiment of the implantable medical electrode, the at least one recess has a geometry selected from the group consisting of a cylinder, a cube, a cuboid, a trapezoid, a square, a circle, a rectangle, and a triangle. This preferred embodiment is a 10^{th} embodiment of the invention, that preferably depends on any of the 1^{st} to 9^{th} embodiments of the invention.

In a preferred embodiment of the implantable medical electrode, the implantable medical electrode comprises a plurality of recesses arranged in a pattern throughout the electrode. This preferred embodiment is an 11^{th} embodiment of the invention, that preferably depends on any of the 1^{st} to 10^{th} embodiments of the invention.

In a preferred embodiment of the implantable medical electrode, the pattern is selected from the group consisting of a grid, axial lines, radial lines. This preferred embodiment is a 12^{th} embodiment of the invention, that preferably depends on any of the 1^{st} to 11^{th} embodiments of the invention.

In a preferred embodiment of the implantable medical electrode, the substrate comprises a material selected from the group consisting of a polymer, a natural rubber, a natural fiber, and a ceramic. This preferred embodiment is a 13^{th} embodiment of the invention, that preferably depends on any of the 1^{st} to 12^{th} embodiments of the invention.

In a preferred embodiment of the implantable medical electrode, the substrate comprises a polymer selected from the group consisting of a polyester, a polyethylene foam, a cellulose rayon non-woven material, polyethylene vinyl acetate, polyurethane, and polyimide. This preferred embodiment is a 14^{th} embodiment of the invention, that preferably depends on any of the 1^{st} to 13^{th} embodiments of the invention.

In a preferred embodiment of the implantable medical electrode, the conducting element comprises a metal selected from the group consisting of platinum, gold, nitinol, and alloys thereof. This preferred embodiment is a 15^{th} embodiment of the invention, that preferably depends on any of the 1^{st} to 14^{th} embodiments of the invention.

A 16^{th} embodiment of the invention is a use of an implantable medical electrode according to the invention, preferably an implantable medical electrode according to any of the 1^{st} to 15^{th} embodiments, in an implantable medical device for stimulation or sensing applications.

A 17^{th} embodiment of the invention is an implantable medical device comprising an implantable medical electrode according to the invention, preferably an implantable medical electrode according to any of the 1^{st} to 15^{th} embodiments of the invention.

An 18^{th} embodiment of the invention is a method of manufacturing an implantable medical electrode for stimulation or sensing, comprising steps of providing an electrically insulating substrate having a substrate surface, wherein the substrate surface comprises at least one recess, wherein the at least one recess comprises: a bottom surface arranged at a first distance to the substrate surface and at least one wall connecting the bottom surface to the substrate surface; and a conducting element arranged in contact with the bottom surface; forming a layer of conductive polymer in contact with the conducting element thus forming an electrical connection with the conducting element when a current is applied, wherein the layer of conductive polymer has a maximum thickness that is less than or equal to the substrate surface.

In a preferred embodiment of the method of manufacturing an implantable medical electrode for stimulation or sensing, the forming step comprises electropolymerization. This preferred embodiment is a 19^{th} embodiment of the invention, that preferably depends on the 18^{th} embodiment of the invention.

In a preferred embodiment of the method of manufacturing an implantable medical electrode for stimulation or sensing, providing the substrate comprises forming a cavity in the substrate by selectively removing material from the substrate. This preferred embodiment is a 20^{th} embodiment of the invention, that preferably depends on any of the 18^{th} to 19^{th} embodiments of the invention.

In a preferred embodiment of the method of manufacturing an implantable medical electrode for stimulation or sensing, the layer of conductive polymer has a maximum thickness that is less than or equal to the substrate surface. This preferred embodiment is a 21^{st} embodiment of the invention, that preferably depends on any of the 18^{th} to 20^{th} embodiments of the invention.

In a preferred embodiment of the method of manufacturing an implantable medical electrode for stimulation or sensing, the substrate is flexible. This preferred embodiment is a 22^{nd} embodiment of the invention, that preferably depends on any of the 18^{th} to 21^{st} embodiments of the invention.

In a preferred embodiment of the method of manufacturing an implantable medical electrode for stimulation or sensing, the layer of conductive polymer protrudes above the substrate surface. This preferred embodiment is a 23^{rd} embodiment of the invention, that preferably depends on any of the 18^{th} to 22^{nd} embodiments of the invention.

In a preferred embodiment of the method of manufacturing an implantable medical electrode for stimulation or sensing, the layer of conductive polymer has a charge storage capacitance ranging from 1 to 400 mC/cm². This preferred embodiment is a 24^{th} embodiment of the invention, that preferably depends on any of the 18^{th} to 23^{rd} embodiments of the invention.

In a preferred embodiment of the method of manufacturing an implantable medical electrode for stimulation or sensing, a connecting element connects the conducting element to a current source. This preferred embodiment is a 25^{th} embodiment of the invention, that preferably depends on any of the 18^{th} to 24^{th} embodiments of the invention.

In a preferred embodiment of the method of manufacturing an implantable medical electrode for stimulation or sensing, the layer of conductive polymer comprises PEDOT. This preferred embodiment is a 26^{th} embodiment of the invention, that preferably depends on any of the 18^{th} to 25^{th} embodiments of the invention.

In a preferred embodiment of the method of manufacturing an implantable medical electrode for stimulation or sensing, the layer of conductive polymer protruding above the substrate surface comprises at least one additional layer or pattern of conductive polymer. This preferred embodiment is a 27^{th} embodiment of the invention, that preferably depends on any of the 18^{th} to 26^{th} embodiments of the invention.

In a preferred embodiment of the method of manufacturing an implantable medical electrode for stimulation or sensing, the at least one recess has a geometry selected from the group consisting of a cylinder, a cube, a cuboid, and a trapezoid. This preferred embodiment is a 28^{th} embodiment of the invention, that preferably depends on any of the 18^{th} to 27^{th} embodiments of the invention.

In a preferred embodiment of the method of manufacturing an implantable medical electrode for stimulation or sensing, the method comprises a plurality of recesses arranged in a pattern. This preferred embodiment is a 29^{th} embodiment of the invention, that preferably depends on any of the 18^{th} to 28^{th} embodiments of the invention.

In a preferred embodiment of the method of manufacturing an implantable medical electrode for stimulation or sensing, the pattern is selected from the group consisting of a grid, axial lines, radial lines. This preferred embodiment is a 30^{th} embodiment of the invention, that preferably depends on the 29^{th} embodiment of the invention.

In a preferred embodiment of the method of manufacturing an implantable medical electrode for stimulation or sensing, the substrate comprises a material selected from the group consisting of a polymer, a natural rubber, a natural fiber, and a ceramic. This preferred embodiment is a 31^{st} embodiment of the invention, that preferably depends on any of the 18^{th} to 30^{th} embodiments of the invention.

In a preferred embodiment of the method of manufacturing an implantable medical electrode for stimulation or sensing, the substrate comprises a polymer selected from the group consisting of a polyester, a polyethylene foam, a cellulose rayon non-woven material, polyethylene vinyl acetate, polyurethane, and polyimide. This preferred embodiment is a 32^{nd} embodiment of the invention, that preferably depends on any of the 18^{th} to 31^{st} embodiments of the invention.

In a preferred embodiment of the method of manufacturing an implantable medical electrode for stimulation or sensing, the conducting element comprises a metal selected from the group consisting of platinum, gold, nitinol, and alloys thereof. This preferred embodiment is a 33^{rd} embodiment of the invention, that preferably depends on any of the 18^{th} to 32^{nd} embodiments of the invention.

A 34^{th} embodiment of the invention is an implantable medical electrode obtainable by a method, according to the invention, of manufacturing an implantable medical electrode for stimulation or sensing, preferably a method as in any of the 18^{th} to 33^{rd} embodiments of the invention.

A 35^{th} embodiment of the invention is a method of treatment, comprising contacting a human patient with the implantable medical electrode according to anyone of the previous embodiments and delivering an electrical signal to said patient.

A 36^{th} embodiment of the invention is a diagnostic method, comprising contacting a human patient with the implantable medical electrode according to anyone of the previous embodiments and detecting an electrical signal from the patient.

In a preferred embodiment of the implantable medical electrode, the layer of conductive polymer protrudes above the substrate surface at a distance of from 0.1 to 2.0 µm. This preferred embodiment is a 37^{th} embodiment of the invention, that preferably depends on the 4^{th} embodiment of the invention.

In a preferred embodiment of the implantable medical electrode, the layer of conductive polymer protruding above the substrate surface has a top view geometry of at least one selected from the group consisting of a circle, a square, a rectangle, a triangle, or a trapezoid. This preferred embodiment is a 38^{th} embodiment of the invention, that preferably depends on any of the 4^{th} or 37^{th} embodiments of the invention.

The embodiments of the invention can be used alone or in combinations with each other.

### BRIEF DESCRIPTION OF SEVERAL VIEWS OF THE DRAWINGS

The following drawings are illustrative of particular embodiments of the present invention and therefore do not limit the scope of the invention. The drawings are not to scale (unless so stated) and are intended for use in conjunction with the explanations in the following detailed description. Embodiments will hereinafter be described in conjunction with the appended drawings wherein like numerals/letters denote like elements.
FIG. 1 is a cross sectional view of an embodiment;
FIG. 2 is a cross sectional view of another embodiment;
FIG. 3 is a cross sectional view of another embodiment;
FIG. 4 is a top view of an embodiment similar to that of FIG. 3;
FIG. 5 is a top view of an alternative embodiment; and
FIG. 6 is a top view of an alternative embodiment shown in FIG. 4.

### DETAILED DESCRIPTION OF THE INVENTION

The following detailed description is exemplary in nature and is not intended to limit the scope, applicability, or configuration of the invention in any way. Rather, the following description provides practical examples, and those skilled in the art will recognize that some of the examples may have suitable alternatives. Examples of constructions, materials, dimensions and fabrication processes are provided for select elements and all other elements employ that which is known by those skilled in the art.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention. The use of the term "comprising" in the specification and the claims includes the narrower language of "consisting essentially of" and "consisting of."

Disclosed herein is an implantable medical electrode for stimulation or sensing, wherein the electrode comprises: an electrically insulating substrate having a substrate surface, wherein the substrate surface comprises at least one recess, wherein the at least one recess comprises: a bottom surface arranged at a first distance to the substrate surface and at least one wall connecting the bottom surface to the substrate surface; a conducting element arranged in contact with the bottom surface; a layer of conductive polymer in contact with the conducting element thus forming an electrical connection with the conducting element when a current is applied.

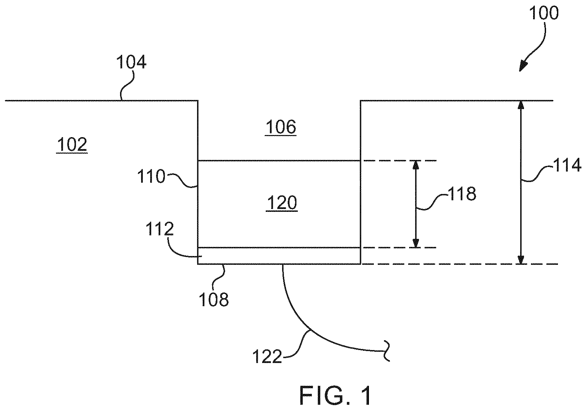

Referring to FIG. 1, implantable medical electrode **100** includes an electrically insulating substrate **102** having a first substrate surface **104** and a second surface (not shown). The electrically insulating substrate **102** can be constructed of any suitable dielectric material or materials, e.g., polymeric, rubber, natural fiber, ceramic, etc. In one or more embodiments, the substrate **102** can include polyester (e.g., MYLAR), polyethylene foam, polyester non-woven materials, cellulose rayon non-woven materials, and polyethylene vinyl acetate films, polyurethane, and polyimide. In an embodiment, the substrate comprises a polymer selected from the group consisting of a polyester, a polyethylene foam, a cellulose rayon non-woven material, polyethylene vinyl acetate, polyurethane, and polyimide.

Electrically insulated substrate **102** comprises at least one recess **106** comprises a bottom surface **108** arranged at a first distance **114** to the substrate surface **104.** The at least one recess **106** may be a hole or via or series of holes or vias arranged in any pattern. In embodiments, the at least one recess **106** may include a grid pattern, a series of parallel lines formed in the substrate, or a series of holes in the form of a grid pattern or in the form of a series of parallel lines. Any pattern can be employed as long as the desired capacitance and pacing impedance is achieved without constraint of physical performance in the electrode design. In some embodiments, the pattern is selected from the group consisting of a grid, axial lines, radial lines.

The at least one recess **106** comprises at least one wall **110** connecting the bottom surface **108** to the substrate surface **104** wherein the geometry of the at least one recess **106** is defined by the at least one wall **110** and may be round or square or any other shape. The geometry of the at least one recess is not particularly important and the at least one recess can have a geometry, for example, selected from the group consisting of a cylinder, a cube, a cuboid, a trapezoid, a square, a circle, a rectangle, and a triangle.

The at least one recess **106** defines a volume. In an exemplary embodiment, the volume defined by each recess is from 500 nm to 50 µm; however, the recess volume typically depends on the geometry of the exposed conductive substrate multiplied by the substrate recess thickness.

As described above, the bottom surface **108** of the at least one recess **106** is located at a first distance **114** from the substrate surface **104.** In some embodiments, the first distance **114** is from 20 to 50 µm, and preferably from 20 to 30 µm. Other exemplary dimensions of the at least one recess include from 3 µm to 10 µm in diameter, and, in some embodiments, from 3 mm to 5 mm in diameter. The dimensions of the at least one recess must be such that they can accommodate an electrodeposition of polymer or conductor material as will be described in more detail below. The at least one recess **106** can be formed in the substrate by any method known to one of ordinary skill in the art to selectively remove material such as, for example, laser cutting.

Still referring to FIG. 1, the at least one recess **106** comprises an electrically conducting element **112** arranged in contact with the bottom surface **108.** Exemplary conducting materials for use as conducting element **112** include conducting metals such as, for example, platinum, gold, nitinol (a nickel-titanium alloy) and alloys thereof.

In embodiments, the electrically conducting element **112** may have a thickness of from 5 µm to 500 µm, preferably from 5 µm to 150 µm. Thickness and size may vary based upon the intended application.

In some embodiments, the conducting element **112** is deposited by a physical vapor deposition (PVD) process as is known to one of ordinary skill in the art such as, for example, sputtering, electrodeposition, lithography, ink deposition, evaporation, ion implantation, or ion beam assisted deposition (IBAD). In other embodiments, a layer of the conductive material forming the electrically conducting element **112** is laminated to a layer of the electrically insulated substrate **102** and the electrically insulated substrate is laser ablated to form the at least one recess until the conductive material forming conducting element **112** is exposed.

Conducting element **112** is electrically connected to a terminal (not shown) which electrically connects the electrode to a control unit (not shown) via connecting element **122.** The control unit may contain electronics to sense various electrical signals of a particular body part such as, for example, the heart or the brain, and also to produce current pulses for delivery to a body part such as, for example, the heart or brain via connecting element **122.**

Still referring to FIG. 1, the at least one recess **106** comprises a layer of conductive polymer **120** in contact with the conducting element **112** thus forming an electrical connection with the conducting element when a current is applied. A conductive polymer mainly comprises an inherently conductive polymer (ICP). The conductive polymer may be biocompatible conductive polymers that are made ionically conductive and that are mechanically stable over a desired period of time, such as, for example, polypyrrole. In further embodiments the conductive polymer may include, for example, polynaphthalene, polythiophene, Nafion, polyethylene oxide, and polyethyldioxythiophene (PEDOT). Other classes of conductive polymers include polyacetylenes, conductive polypyrrole polystyrene sulfonate, polythiophenes (PT), and polyanilines. Conductive polymers may also include EHPT (poly(3-(2-ethylhexyl)thiophene), ionomers (e.g., NAFION®), poly(3,4 ethylene dioxythiophene) (PEDOT) and PEDOT polystyrene sulfonate (PSS/PEDOT), polyacrylamide, and polyvinylpyrrolidone. The conductive polymers are biocompatible (e.g., the polymers are not toxic or injurious to living tissue).

Dopants may also be used with the conductive polymers. Doping may enhance the conductivity of a polymer and provide a lower energy threshold for conductivity. Dopants may also help to specifically control the conductivity characteristics. There are many methods and materials useful in doping that may be known to those skilled in the art. Doping materials can include, but are not limited to chloride, polystyrene sulfonate (PSS), dodecylbenzenesulfonate, polystyrenesulfonate, naphthalene sulfonate, and lithium perchlorate.

Conductive polymer layer **120** is preferably deposited by an electrodeposition process (also referred to herein as "electropolymerization") as is known to those skilled in the art. As used herein "electrodeposition" is the deposition of a material that occurs upon the application of an electrical potential between two conductive materials (or electrodes) within a liquid medium containing charged species. In various embodiments, materials are electrodeposited at the anode (i.e., the electrode where monomer oxidation takes place). A typical apparatus for carrying out electrodeposition includes the following: an anode, a cathode and, frequently, a reference electrode, each separated by an electrolyte (e.g., an ion containing solution), as well as a potentiostat which monitors/sets the voltages/currents at the various electrodes. Electrodeposition can be carried out under a variety of electrochemical conditions including the following, among others: (a) constant current, (b) constant voltage, (c) current scan/sweep, e.g., via a single or multiple scans/sweeps, (d) voltage scan/sweep, e.g., via a single or multiple scans/sweeps, (e) current square waves or other current pulse wave forms, (f) voltage square waves or other voltage pulse wave forms, and (g) a combination of different current and voltage parameters.

The electrodeposition process can be controlled to deposit layers **120** of conductive polymer varying in thickness. For example, in FIG. 1, the thickness of conductive polymer layer **120** is shown as reference number **118,** which is less than (i.e., below) the level of the substrate surface **104,** which is typically an outer surface of the electrode **100.** In such embodiments, recess **106** above the conductive polymer layer as shown in FIG. 1 may serve as a site for tissue to grow into once implanted to better anchor the electrode at the point of use in a patient's body.

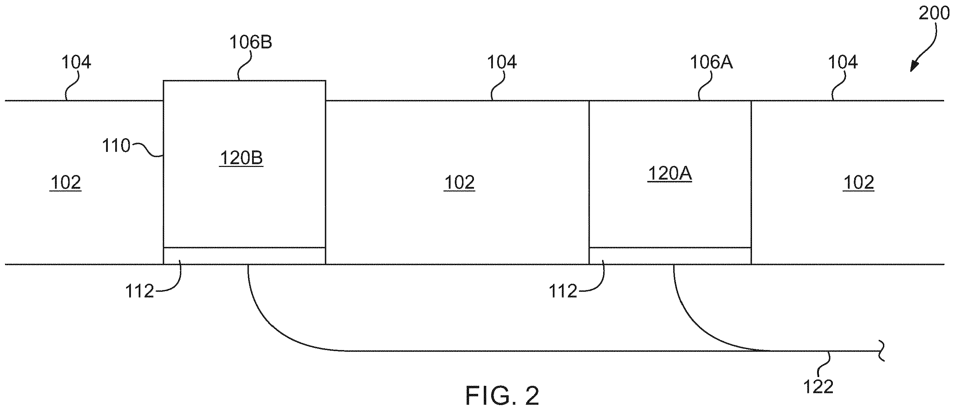

Referring now to FIG. 2, another embodiment is shown for purposes of illustration of the thickness of conductive polymer layer **120.** Electrode **200** comprises at least two recesses **106A** and **106B** shown comprising conductive element **112** and conductive polymer layer **120A** and **120B.** The thickness of conductive polymer layer **120A** is even with the surface **104** of the electrically insulating substrate **102.** The thickness of conductive polymer layer **120B** protrudes above the surface **104** of the electrically insulating substrate **102.** As will be described below, is some embodiments the surface **104** may have some overlap with the conductive polymer laterally from the recess, however, in no embodiment is the surface **104** fully covered with a conductive polymer as a layer.

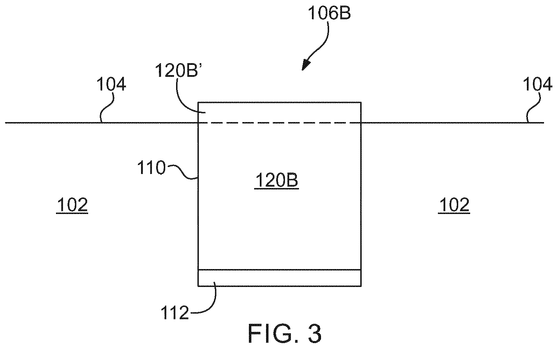

The thickness of conductive polymer layer **120B** can be achieved by use of a mask such as a photolithography mask. The thickness of the mask can, for example, be equal to the distance from which conductive polymer layer **120B** protrudes above surface **104.** Also, by use of a mask or more than one mask, the surface of conductive polymer layer **120B** can have varying geometries or layers. For example, referring to FIG. 3, an embodiment is shown wherein protruding portion **120B'** of layer **120B** of conductive polymer layer **120** is shown. Protruding portion **120B'** may have the same or a different geometry relative to the geometry of the recess or relative to conductive polymer **120B** on which it is located. For example, the geometry of protruding portion **120B'** can be a circle, a square, a rectangle, a triangle, or a trapezoid from a top view. Preferably, **120B** is completely integrated with the underlying conductive polymer **120B** and, thus, is an extension of conductive polymer layer **120B** and not a separate layer having an interface between **120B** and **120 B'.** In the exemplary embodiment shown in FIG. 3, conductive polymer protruding portion **120B'** has the same dimensions as the recess from which it extends and there is no lateral growth due to the use of, for example, a mask while forming the layer.

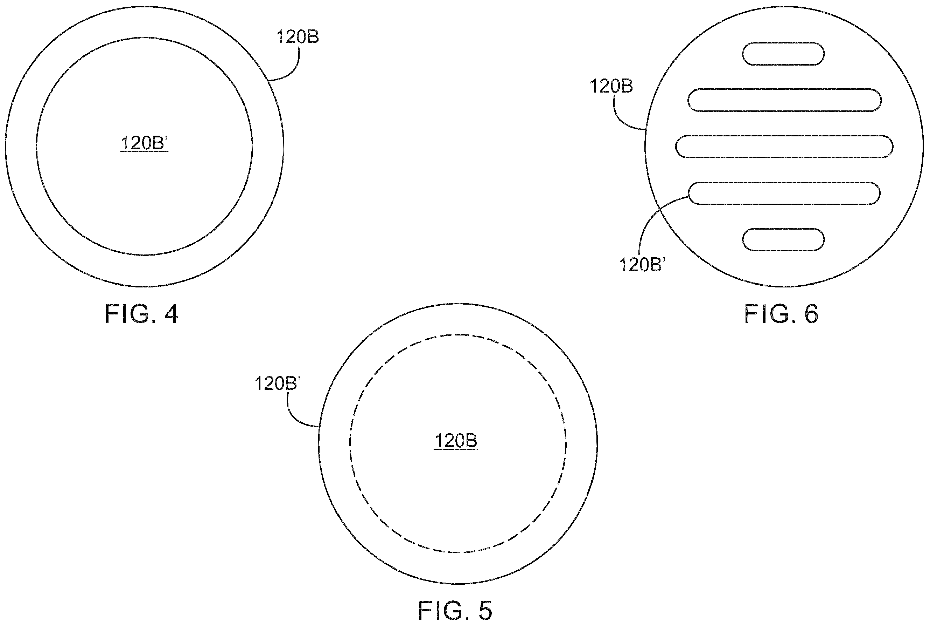

In another embodiment shown in FIG. 4 as a top view of an embodiment similar to that shown of FIG. 3, conductive polymer protruding portion **120B'** is solid and has a smaller diameter than conductive polymer layer **120B.** In another embodiment shown in FIG. 6, which is also a top view of an alternative embodiment shown in FIG. 4, conductive polymer layer **120B'** is a series of linear protrusions. One skilled in the art will recognize that the same additional layers can be added to the surface of conductive polymer layer **120A** in FIG. 2.

In yet another embodiment shown in FIG. 5, which, for illustration, is a top view of an embodiment where the surface of conductive polymer protruding portion **120B'** extends beyond the recess **106** (shown as a dotted line) and onto substrate surface **104** (not shown) to increase the tissue contact surface. In this embodiment, here again, conductive polymer protruding portion **120B'** and **120B** are integrated and are one body. One of ordinary skill in the art will appreciate that the use of a mask in depositing the conductive polymer protruding portion **120B'** will prevent uncontrolled lateral growth. Patterns may be formed in this or any other embodiment by use of the appropriate mask or by, for example, laser ablation.

Preferably, the thickness of the conductive polymer protruding portion **120B'** above substrate surface **104** as shown in, for example, FIGS. 2 to 6 is from 0.1 to 3 µm above substrate surface **104,** and in some embodiments from 0.1 to 2.0 µm, and in other embodiments from 1.0 to 2.0 µm. If the thickness is much more, the risk of damage during application in a body increases.

The ability to vary the thickness of conductive polymer layer **120** as shown in **120, 120A** and **120B** in FIGS. 1 and 2, has the advantage of varying the charge storage capacitance (CSC) of the layer based on varying usage needs. For example, a thicker conductive polymer layer **120** will allow a greater CSC accumulation on the electrode which may be more suitable for neurostimulation applications. In embodiments, the charge storage capacitance of the conductive polymer layer ranges from 1 to 400 mC/cm².

High electrode capacitance and therefore low electrode impedance and a high degree of biocompatibility are of importance for the usage value of an implantable stimulation electrode, particularly one which is intended for long-term use on a tissue stimulator having an exhaustible energy source and which therefore must contribute to the minimal energy consumption.

The implantable recessed electrodes disclosed herein are particularly useful for the stimulation of bodily tissue, particularly for use in pacemakers, defibrillators, and bone stimulators or neurostimulators. Thus, disclosed herein is an implantable medical device comprising the implantable medical electrodes disclosed herein. This includes, for example, a tissue stimulator having an implantable electrode of the invention as described above. Typically, such an implantable tissue stimulator has electrical power supply means and means for applying electrical stimulation pulses to the electrode using electrical power from the power supply means. The power supply means may be electrical power storage means such as a battery, and/or means for receiving electrical power transmitted from outside the body, e.g., by radio frequency coupling. The implantable tissue stimulator may be, for example, a pacemaker, a defibrillator, a bone stimulator or a neuro-stimulator.

Thus, in embodiments, disclosed here is a method of treating a patient in need thereof, comprising contacting a human patient with the implantable medical electrode disclosed herein and delivering an electrical signal to the patient or receiving an electrical signal from the patient. In embodiments wherein an electrical signal is received from a patient, embodiments include a diagnostic method comprising contacting a human patient with the implantable medical electrode disclosed herein and receiving an electrical signal from the patient.

Also disclosed is a method of manufacturing an implantable medical electrode for stimulation or sensing, comprising steps of providing an electrically insulating substrate having a substrate surface, wherein the substrate surface comprises at least one recess, wherein the at least one recess comprises: a bottom surface arranged at a first distance to the substrate surface and at least one wall connecting the bottom surface to the substrate surface; and a conducting element arranged in contact with the bottom surface; and forming a layer of conductive polymer in contact with the conducting element thus forming an electrical connection with the conducting element when a current is applied, wherein the layer of conductive polymer has a maximum thickness that is less than or equal to the substrate surface.

Techniques for this procedure of electropolymerisation are known and are described in the prior art. In the forming step, the layer of conductive polymer is deposited by electropolymerisation, by reacting the respective monomers (e.g., polypyrrole, substituted polypyrrole, thiophene, substituted polythiophene, etc.).

In the foregoing detailed description, the invention has been described with reference to specific embodiments. However, it will be appreciated that various modifications and changes can be made without departing from the scope of the invention as set forth in the appended claims.

## Claims

1. An implantable medical electrode for stimulation or sensing, wherein the electrode comprises:
an electrically insulating substrate having a substrate surface, wherein the substrate surface comprises at least one recess, wherein the at least one recess comprises:
a bottom surface arranged at a first distance to the substrate surface and at least one wall connecting the bottom surface to the substrate surface;
a conducting element arranged in contact with the bottom surface;
a layer of conductive polymer in contact with the conducting element thus forming an electrical connection with the conducting element when a current is applied.

2. The implantable medical electrode according to claim 1 wherein the layer of conductive polymer has a maximum thickness that is less than or equal to the substrate surface.

3. The implantable medical electrode according to claim 1 or claim 2 wherein the substrate is flexible.

4. The implantable medical electrode as in claim 1 wherein the layer of conductive polymer protrudes above the substrate surface.

5. The implantable medical electrode as in any one of the preceding claims wherein the layer of conductive polymer has a charge storage capacitance ranging from 1 to 400 mC/cm².

6. The implantable medical electrode as in any one of the preceding claims wherein the layer of conductive polymer comprises PEDOT.

7. The implantable medical electrode of claim 4 wherein the layer of conductive polymer protruding above the substrate surface comprises a pattern of lines.

8. The implantable medical electrode of claim 4 wherein the layer of conductive polymer protruding above the substrate surface also extends laterally beyond the recess.

9. The implantable medical electrode as in any one of the preceding claims wherein the conducting element comprises a metal selected from the group consisting of platinum, gold, nitinol, and alloys thereof.

10. A use of the implantable medical electrode according to any one of the preceding claims in an implantable medical device for stimulation or sensing applications.

11. An implantable medical device comprising the implantable medical electrode as in any one of claims 1 to 9.

12. A method of manufacturing an implantable medical electrode for stimulation or sensing, comprising steps of
a. providing an electrically insulating substrate having a substrate surface, wherein the substrate surface comprises at least one recess, wherein the at least one recess comprises:
a bottom surface arranged at a first distance to the substrate surface and at least one wall connecting the bottom surface to the substrate surface; and a conducting element arranged in contact with the bottom surface;
b. forming a layer of conductive polymer in contact with the conducting element thus forming an electrical connection with the conducting element when a current is applied, wherein the layer of conductive polymer has a maximum thickness that is less than or equal to the substrate surface.

13. The method of claim 12 wherein the forming step comprises electropolymerization.

14. The method as in claim 12 or 13 wherein providing the substrate comprises forming a cavity in the substrate by selectively removing material from the substrate.

15. The method as in any one of claims 12 to 14 wherein the substrate is flexible.

16. The method as in any one of claims 12 to 15 wherein the layer of conductive polymer has a charge storage capacitance ranging from 1 to 400 mC/cm².

17. The method as in any one of claims 12 to 16 wherein the layer of conductive polymer comprises PEDOT.

18. The method as in any one of claims 12 to 17 wherein the layer of conductive polymer protruding above the substrate surface comprises at least one additional layer or pattern of conductive polymer.

19. The method as in any one of claims 12 to 18 wherein the conducting element comprises a metal selected from the group consisting of platinum, gold, nitinol, and alloys thereof.

20. An implantable medical electrode obtainable by the method as in any one of claims 12 to 19.
